# EUROPEAN PATENT APPLICATION

(11) **EP 0 650 964 A1**
(43) Date of publication of application: **03.05.1995**
(21) Application number: 94203088.3
(22) Date of filing: 25.10.1994
(51) Int. Cl.: C07D 311/18, A61K 31/495

(54) **1 2H-1-benzopyran-2-one-8-yl -piperazine derivatives**

(30) Priority: 02.11.1993 EP 93203058
(71) Applicant: DUPHAR INTERNATIONAL RESEARCH B.V, NL-1381 CP Weesp (NL)
(72) Inventor: van Steen, Bartholomeus Johannes, NL-1380 AC Weesp (NL); Hartog, Jan, NL-1380 AC Weesp (NL); van der Heyden, Johannes Antonius Maria, NL-1380 AC Weesp (NL); Schipper, Jacques, NL-1380 AC Weesp (NL)
(74) Representative: Muis, Maarten

(57) **Abstract**

The invention relates to a group of new 1-[2H-1-benzopyran-2-one-8-yl]-piperazine derivatives having interesting pharmacological properties due to 5-HT_{1A} agonism and 5-HT_{1D}-antagonism.

The compounds have the general formula (1) 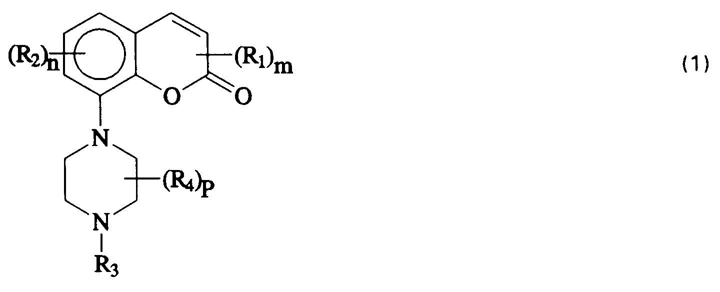
wherein
- R₁ is alkyl (1-4C), alkoxy (1-4C), hydroxy, alkoxy(1-4C)alkyl(1-4C), pyrrolidinyl, piperidinyl, morpholinyl, halogen, cyano, trifluoromethyl, amino, or mono- or disubstituted amino wherein the substituents are alkyl (1-4C), or alkyl(1-4C)carbonyl,
- m has the value 0, 1 or 2,
- R₂ is alkyl (1-4C), alkoxy (1-4C), halogen or trifluoromethyl,
- n is 0 or 1, on the understanding that m + n is at least 1,
- R₃ is hydrogen, alkyl (1-3C) or alkenyl (2-3C),
- R₄ is alkyl (1-4C), and
- p has the value 0, 1 or 2, and pharmaceutically acceptable acid addition salts thereof.

The compounds can be used for the treatment of affections or diseases of the central nervous system caused by disturbances of the serotonergic transmission.

## Description

The invention relates to a group of new 1-[2H-1-benzopyran-2-one-8-yl]-piperazine derivatives having interesting pharmacological properties.

It has been shown in compounds of the hetero(bicyclic)arylpiperazine class which are clinically effective antidepressants and anxiolytics, such as for example buspirone, that their affinity for the 5-HT_{1A} receptor is related to their pharmacological and clinical activity.

Surprisingly it has now been found that a new group of 1-[2H-1-benzopyran-2-one-8-yl]-piperazine dervatives of the formula (1) 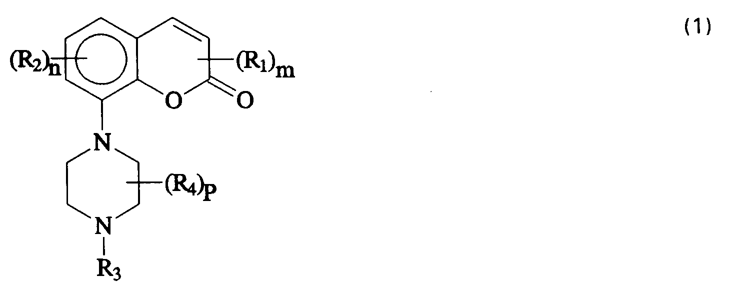
wherein
- R₁ is alkyl (1-4C), alkoxy (1-4C), hydroxy, alkoxy(1-4C)alkyl(1-4C), pyrrolidinyl, piperidinyl, morpholinyl, halogen, cyano, trifluoromethyl, amino, or mono- or disubstituted amino wherein the substituents are alkyl (1-4C), or alkyl(1-4C)carbonyl,
- m has the value 0, 1 or 2,
- R₂ is alkyl (1-4C), alkoxy (1-4C), halogen or trifluoromethyl,
- n is 0 or 1, on the understanding that m + n is at least 1,
- R₃ is hydrogen, alkyl (1-3C) or alkenyl (2-3C),
- R₄ is alkyl (1-4C), and
- p has the value 0,1 or 2,
and pharmaceutically acceptable acids addition salts thereof act on the central nervous system by binding to 5-HT receptors. The alkyl and alkoxy groups represented by R₁ to R₄ can be straight or branched.

More particularly it has been found that these compounds bind to subtypes of the 5-HT receptor i.e. 5-HT_{1A} and 5-HT_{1D} subtype receptors. Furthermore, the compounds of the invention bind to these receptors in greater extent than they bind to other receptors of the 5-HT-type and other central nervous receptors, such as dopaminergic and adrenergic receptors.

Known clinical effective antidepressants and anxiolytics belonging to the hetero(bicyclic)arylpiperazine class which bind to the 5-HT_{1A} receptor all have a structural similarity with respect to the substituents present on the aliphatic amino group. It is well established that their affinity for the 5-HT_{1A} receptor is predominantly caused by the interaction of these substituents with the 5-HT_{1A} receptor.

Surprisingly, it has now been found that the compounds of the invention have an extremely high affinity (even in the subnanomolar range) for the 5-HT_{1A} receptor although they are not substituted at the aliphatic nitrogen atom. In spite of this, the compounds of the invention act as strong 5-HT_{1A} agonists in functional models.

For this reason it is very surprisingly that compounds as defined above have an extremely high affinity for the 5-HT_{1A} receptor although no large substituent is present on this aliphatic nitrogen atom.

Furthermore, it has been found that besides a high affinity for the 5-HT_{1A} receptor many of the present compounds display a nanomolar affinity for 5-HT_{1D} receptors and act as 5-HT_{1D} antagonists.

The presence of 5-HT_{1D} antagonism is of therapeutic value. 5-HT_{1D} receptors are located presynaptically on the nerve terminal and have a negative modulatory influence on the release of 5-HT. Therefore, blockade of these receptors enhances the release of 5-HT from its terminals. A similar effect can be achieved by blocking the reuptake of this monoamine (which is the mechanism of action of antidepressants such as fluvoxamine and fluoxetine).

The presence of 5-HT_{1A} agonism results in an antidepressant activity in man; the additional presence of presynaptic 5-HT_{1D} antagonism will result in a similar effect as observed after administration of 5-HT reuptake inhibitors which have proven to be clinically active antidepressants. When 5-HT_{1D} antagonism is combined with 5-HT_{1D} agonism the antidepressant activity therefore is strengthened.

The compounds of the invention combine the favourable mechanisms of 5-HT_{1A} agonism and 5-HT_{1D} antagonism.

Further it is known from EP 0189612 that a group of heterobicyclic piperazine derivatives has psychotropic activity.

The 5-HT_{1D} antagonistic activity component is not present in the compounds described in EP. 0189612, not even in the most closely related compound, i.e. 1-[2H-1-benzopyran-2-one-8-yl] piperazine.

Especially preferred are the compounds having formula (1) wherein (R₂)ₙ and (R₄)ₚ are hydrogen, R₃ has the above mentioned meanings, and (R₁)ₘ is a substituent at position 3 selected from the group consisting of pyrrolidinyl, piperidinyl, morpholinyl, amino and mono- or disubstituted amino wherein the substituents are alkyl (1-4C) or alkyl (1-4C) carbonyl.

More particularly the compound 1-[3-amino-2H-1-benzopyran-2-one-8-yl]-piperazine and the salts thereof is preferred, i.e. the compound of formula (1) wherein (R₁)ₘ is 3-NH₂, (R₂)ₙ R₃ and (R₄)ₚ are hydrogen.

When a centre of chirality is present in a compound of the formula (1) the invention relates both to the racemate and the individual enantiomers thereof.

Suitable acids with which the compounds can form pharmaceutically acceptable acid addition salts are for example hydrochloric acid, sulphuric acid, phosphoric acid, nitric acid, and organic acids such as citric acid, fumaric acid, maleic acid, tartaric acid, acetic acid, benzoic acid, p-toluene-sulphonic acid, methanesulphonic acid and naphtalenesulphonic acid.

The compounds having formula (1) can be used for the treatment of affections or diseases of the central nervous system caused by disturbances of the serotonergic transmission, for example psychoses, aggression, anxiety disorders, autism, vertigo, disturbances of cognition or memory, and in particular for the treatment of depression.

The compounds of the invention can be brought into forms suitable for administration by means of usual processes using auxiliary substances such as liquid and solid carrier materials.
Pharmacological test procedures:
1) Measurement of 5-HT_{1A} receptor affinity was determined by using ³H-8-OH-DPAT as a ligand according to the method described by Gozlan et al, Identification of presynaptic serotonin autoreceptors using a new ligand: ³H-PAT, Nature, 1983, 305, 140-142.
2) Measurement of 5-HT_{1A} agonistic activity in vitro
   With the introduction of a cloned human 5-HT_{1A} receptor in CHO host cells, which is negatively coupled to adenylate cyclase, is it possible to investigate agonism/antagonism of compounds without the involvement of other receptor subtypes (Raymond et al. Arch. Pharmacol, 1992, 346, 127-137; Pauwels et al., Biochem. Pharm., 1993, 45, 375-383). The inhibition of cAMP was measured after stimulation with forskolin with or without the test drug according to the method described by Salomon et al. Analytical Biochemistry, 1974, 58, 541-548.
3) Measurement of 5-HT_{1D} receptor affinity
   The affinity of a drug for the 5-HT_{1D} receptor was determined using ³H-5-HT as a ligand, using methods described by Heuring and Peroutka, J. Neuroscience, 1987, 7, 894-903.
4) Measurement of 5-HT_{1D} antagonistic activity in vitro
   The modulatory effects of drugs on serotonergic neurotransmission were investigated in vitro. It has been shown that activation of presynaptic 5-HT receptors causes a strong inhibition of [³H]-serotonin release from nerve terminals in the cortex of the rat (Göthert, Naunyn-Schmiedberg's Arch. Pharmacol., 1980, 314, 223). This study was performed using the cortex of the guinea-pig, which was found to express presynaptic 5-HT receptors identical to those present in human brain (5-HT_{1D}) (Hoyer et al., Trends Pharmacol. Sci., 1989, 10, 130). The potassium-evoked release of [³H]-5-HT from guinea-pig cortex slices, inhibited by 5-HT, was measured in absence and presence of the test drugs. The experiments were performed using the method described by Frankenhuyzen and Mulder, Eur. J. Pharmacol, 1982, 78, 91.
5) Measurement antidepressant forced-swim activity
   The rat forced-swim test was introduced by Porsolt et al., Eur. J. Pharmacol., 1978, 47, 379-391 as an animal model sensitive to antidepressant treatments. Briefly, when rats are forced to swim in a confined inescapable space, they tend to become immobile after initial vigorous escape activity. The animals' immobility was qualified by Porsolt as 'behavioural despair'. Tricyclic antidepressants, monoamine-oxidase inhibitors, atypical antidepressants as well as electroconvulsive shocks reduce the immobility in this forced swim test (also see; Duncan et al. J. Pharmacol. Exp. Ther., 1985, 234, 402-408). This test is now generally considered to have a good predictive value for antidepressant activity in man (Willner, Psychopharmacol., 1984, 83, 1-16). The drugs were tested using the protocol described by Porsolt et al. (1978).

The new compounds having formula (1) and their salts can be prepared according to method known per se for structurally related known compounds, for example by using the methods as described in EP 0189612.

A suitable reaction scheme is the following:
Reaction of a compound of the formula (2) 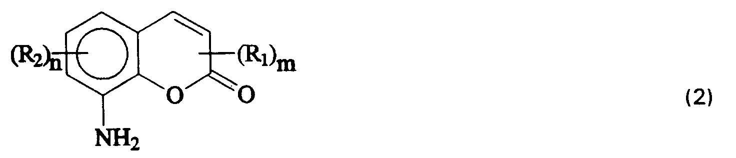
with a compound of the formula (3), or a salt thereof 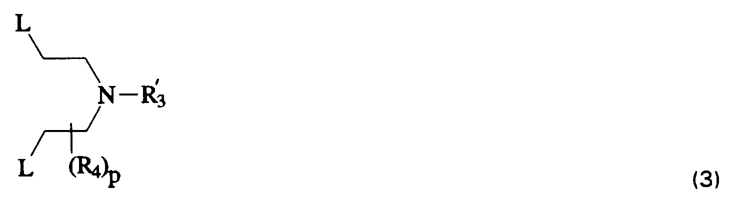
in which formulae R₁, R₂,R₄, m, n and p have the meanings given above, and L is a so-called leaving group, for example a halogen atom, or sulfonate group, and R₃ has the meanings given for R₃ or represents a group which protects the nitrogen atom, for example the benzyl group.

This reaction may be carried out both in an inert apolar organic solvent, and in a protic polar solvent. Examples of suitable solvents are chlorobenzene, toluene, pyridine, acetonitrile, lower aliphatic alcohols, for example, ethanol, propanol and butanol. In order to bind the releasing acid, an acid binder, for example NaHCO₃ or K₂CO₃ or an organic base, for example, triethylamine, may be used.

It is sometimes necessary or desired in this mode of preparation first to replace the hydrogen atom at the nitrogen atom in the starting material of formula (3) by a protective group, for example, the benzyl group, an aryloxycarbonyl group or alkoxycarbonyl group having 1-4 C-atoms in the alkoxy group. Said protective group can then be removed from the resulting final product by means of the methods conventionally used for this purpose, for example, by catalytic hydrogenation or by acid hydrolysis. Conventional solvents are lower aliphatic alcohols and aliphatic esters thereof or aqueous mineral acid. The reactions are carried out at temperatures between room temperature and reflux temperature of the solvent used.

The starting compounds of the formula (2) can be prepared by using chemical reactions known for the synthesis of coumarins. The synthesis of compounds (2) wherein (R₁)ₘ is the 3-amino group can be carried out as follows (e.g. see J. Org. Chem., 1960,25, 1817-1819):
Reaction of substituted nitrosalicylaldehyde derivative (4) 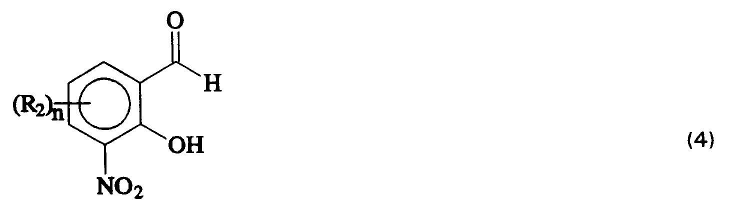
with N-acetylglycine (CH₃CONHCH₂COOH). This reaction can be carried out by heating a mixture of substituted-3-nitrosalicylaldehyde, N-acetylglycine, sodium acetate and acetic acid anhydride at 100°C for 1-2 hours. The reaction mixture is then cooled down and the solid filtered off. The crude product can be further purified by column chromatography or recrystalization.
The nitro group in the so-obtained compound of the formula (5) 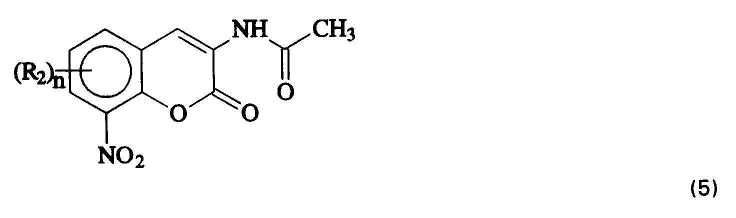
can be reduced to the desired amino group by means of suitable reduction reactions. A suitable procedure for this reduction is the following: the substituted-8-nitrocoumarin is added to a stirred mixture of iron powder, ethanol, water and 2N HCl at 55-60°C. The stirring is continued for 3 hours and the reaction mixture cooled down and filtered over hyflo. An organic solvent is added, e.g. dichloromethane, and the mixture is extracted. The organic solvent is removed in vacuo and the crude product is converted into its hydrochloride salt and further purified by recrystallization.

The compounds having formula (1) can also be prepared from a compound of the formula (6) 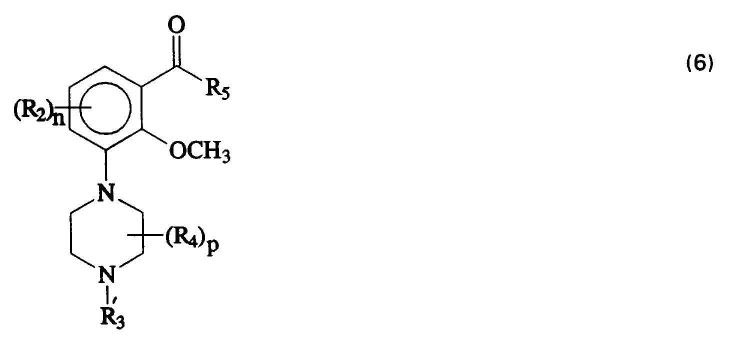
wherein R₂, R'₃, R₄, n and p have the above meanings and R₅ is hydrogen or methyl, according to methods known for the synthesis of coumarins.

The starting comppounds of the formule (6) can be prepared by reacting a compound of the formula (7) 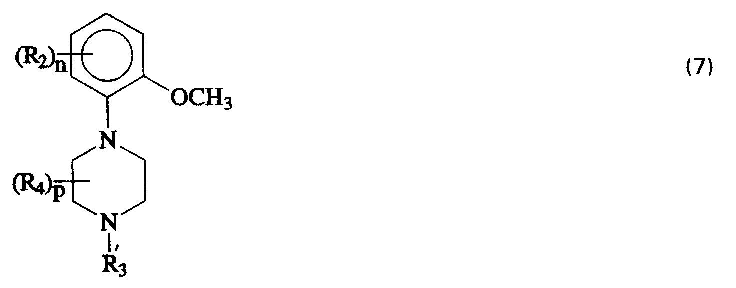
with butyllithium and dimethylformamide or N-methoxy-N-methylacetamide according to standard procedures.

The compounds having formula (7) can be obtained as described in EP. 0556889.

The preparation of the compounds will now be described in the following specific examples. It is noticed that intermediates 6-8, 19 and 24 are also compounds having formula (1) as defined above.

### Intermediate 1

### 4-benzyl-1-[3-acetamide-2H-1-benzopyran-2-one-8-yl]-piperazine, hydrochloride

This intermediate was prepared according to the method described for 1-[3-methyl-2H-1-benzopyran-2-one-8-yl]-piperazine hydrochloride (Example 1) using 8-amino-3-acetamide-2H-1-benzopyran-2-one hydrochloride (3.50 g, 13.7 mmol), N-benzyl-bis(2-chloroethyl)amine hydrochloride (4.06 g, 15.1 mmol) and p-toluenesulfonic acid monohydrate (2.60 g, 13.7 mmol) in 220 ml of chlorobenzene. The mixture was refluxed for 4 days. The crude product was purified by recrystallization from absolute ethanol. Yield : 4.10 g (72%).

### Intermediate 2

### 4-benzyl-1-[3-hydroxy-2H-1-benzopyran-2-one-8-yl]-piperazine, hydrochloride

Intermediate 1 (4.00 g, 9.66 mmol) was refluxed in 100 ml of 1 N hydrochloride for 2 hours. The solution was neutralized by addition of 25% ammonia and the solvent was removed in vacuo. The residue was purified by column chromatography (SiO₂ // CH₂Cl₂: MeOH = 90 : 10). Yield : 2.40 g (74%) , m.p. 80-95°C.

### Intermediate 3

### 4-benzyl-1-[3-methoxy-2H-1-benzopyran-2-one-8yl]-piperazine, hydrochloride

To a stirred solution of 8-amino-3-methoxy-2H-1-benzopyran-2-one (1.35 g, 5.93 mmol), N-benzyl-bis-(2-chloroethyl)amine hydrochloride (1.60 g, 5.93 mmol) was added p-toluenesulfonic acid monohydrate (1.10 g, 5.93 mmol). The reaction mixture was heated for 18 hours under a nitrogen atmosphere using a Dean-Stark water separator. The dark solution was cooled down and the solvent evaporated in vacuo. The residue was purified by column chromatography (SiO₂ // CH₂Cl₂ : MeOH = 95 : 5) and converted into its hydrochloride salt. Yield : 1.60 g (71%)

### Intermediate 4

### 4-benzyl-1-[3-methylamino-2H-1-benzopyran-2-one-8-yl]-piperazine hydrochloride

A mixture of 8-amino-3-methylamino-2H-1-benzopyran-2-one hydrochloride (1.75 g, 7.72 mmol), N-benzyl-bis-(2-chloroethyl)amine hydrochloride (2.07 g, 7.72 mmol) and p-toluenesulfonic acid monohydrate (1.47 g, 7.72 mmol) in 285 ml of chlorobenzene was heated at 135°C under a nitrogen atmosphere for 18 hours. The reaction mixture was cooled down and the solvent was evaporated in vacuo. The residue was purified by column chromatography (SiO₂ // CH₂Cl₂ : MeOH = 95 : 5) and converted into its hydrochloride salt. Yield : 1.20 g (45%), m.p. 229-31°C.

### Intermediate 5

### 4-benzyl-1-[3-amino-2H-1-benzopyran-2-one-8-yl]-piperazine, hydrochloride

This intermediate was prepared from 4-benzyl-1-[3-acetamide-2H-1-benzopyran-2-one-8-yl]-piperazine hydrochloride (Intermediate 1) (4.00 g, 9.68 mmol) which was hydrolyzed at 60°C for 3 hours in a mixture of 200 ml of 50% H₂SO₄ and 200 ml of acetic acid. The reaction mixture was cooled down and then neutralized with sodium bicarbonate. The crude product was filtered off, washed with water and converted into its hydrochloride salt. Recrystallization from ethanol gave 3.30 g (92%) of 4-benzyl-1-[3-amino-2H-1-benzopyran-2-one-8-yl]-piperazine hydrochloride, m.p. 264-7°C.

### Intermediate 6

### 4-methyl-1-[3-acetamido-2H-1-benzopyran-2-one-8-yl]-piperazine

To a stirred suspension of 8-amino-1-(3-acetamido-2H-1-benzopyran-2-one)piperazine (3.00 g, 13.8 mmol) in 80 ml of p-xylene and 80 ml of chlorobenzene was added N-methyl-bis-(2-chloroethyl)amine (1.70 g, 14.2 mmol) and p-toluenesulfonic acid monohydrate (2.61 g). The mixture was heated at 160°C for 18 hours while stirring under a nitrogen atmosphere and using a Dean-Stark water-separator. The reaction mixture was cooled down and concentrated in vacuo. Purification of the dark residue by column chromatography (SiO₂) (CH₂Cl₂ : MeOH : NH₄OH = 92: 7.5 : 0.5) gave the title compound.

### Intermediate 7

### 4-isopropyl-1-[3-acetamido-2H-1-benzopyran-2-one-8-yl]-piperazine

This intermediate was prepared from 1-[3-acetamide-2H-1-benzopyran-2-one-8-yl]-piperazine, hydrochloride (Example 4) which was alkylated with isopropylbromide using diisopropylethylamine and sodiumiodide in acetonitrile according to standard procedures.

### Intermediate 8

### 4-allyl-1-[3-acetamido-2H-1-benzopyran-2-one-8-yl]-piperazine

This intermediate was prepared by to the method described for 4-isopropyl-1-[3-acetamido-2H-1-benzopyran-2-one-8-yl]-piperazine (Intermediate 7) using allylbromide.

### Intermediate 9

### 8-nitro-3-n-propyl-2H-1-benzopyran-2-one

To a mixture of 3-nitrosalicylaldehyde (8.35 g, 0.05 mol), pentanoic acid (5.00 g, 0.05 mol) and pentanoic anhydride (18.6 g, 0.10 mol) was added triethylamine (7.0 ml, 0.05 mol). The reaction mixture was heated at 150°C for 5 hours, cooled down and neutralized with a 5% sodiumbicarbonate solution. The mixture was extracted with dichloromethane (3x) and the combined organic layers washed with 5% sodiumbicarbonate and water, dried and the solvent was removed in vacuo. The dark residue was purified by column chromatography (SiO₂ // methyl-t-butylether). Yield 4.7 g (40%) of the title compound.

### Intermediate 10

### 8-amino-3-n-propyl-2H-1-benzopyran-2-one

This intermediate was prepared according to standard procedures : Intermediate 9 (4.00 g, 17.2 mmol) was added at 55°C to a stirred solution of 9.5 g iron powder in 70 ml of water, 70 ml of methanol and 1 ml concentrated hydrochloric acid. Stirring was continued for 1 hour and the reaction mixture was cooled down and extracted with dichloromethane. The organic layer was separated and washed with water, dried and the solvent removed in vacuo. The so obtained crude product was pure enough for the next reaction of Example 15.

### Intermediate 11

### 4-benzyl-1-(2-methoxyphenyl)piperazine

To a stirred suspension of o-methoxyphenylpiperazine (100.0 g, 440 mmol) and potassiumcarbonate (121.0 g, 875 mmol) in 800 ml of dry acetonitrile was added benzylbromide (57 ml, 480 mmol). The reaction mixture was heated at reflux temperature for 1 night and cooled down, and the solvent removed in vacuo. The residue was taken up in ethylacetate and 2N sodiumhydroxide and extracted. The organic layers were combined, dried and the solvent removed. The crude product was further purified by flash column chromatography (SiO₂ // diethylether : pentane = 1: 3). Yield 104.0 g (84%) of the title compound.

### Intermediate 12

### 4-benzyl-1-(3-acetyl-2-methoxyphenyl)piperazine

To a stirred solution of Intermediate 11 (8.55 g, 30.3 mmol) in 200 ml of dry tetrahydrofuran at -78°C was added sec-butyllithium (93 ml, 121 mmol (1.3 M)) under a nitrogen atmosphere. The reaction mixture was warmed up to reflux temperature and stirring was continued at +60°C for ½ hour or until no more starting material was detectable by TLC. The reaction mixture was cooled to -70°C and N-methoxy-N-methylacetamide (3.75 g, 36.4 mmol) was added. Stirring was continued for ½ hour at ambient temperature before 5% sodiumbicarbonate and ethylacetate were added. The mixture was extracted with ethylacetate and the combined organic layers were washed with water, dried and the solvent was removed in vacuo. The crude product was further purified by column chromatography (SiO₂ // diethylether : hexane = 1 : 1). Yield 4.32 g (44%) of the title compound.

### Intermediate 13

### 4-benzyl-1-(3-acetyl-2-hydroxyphenyl)piperazine

The title compound was prepared by heating Intermediate 12 (6.37 g, 19.7 mmol) in 100 ml of 48% hydrobromic acid for 6 hours at 110°C. The reaction mixture was cooled down, poured into 2N sodiumhydroxide and ethylacetate and extracted with ethylacetate at pH 8. The combined organic layers were dried and the solvent was removed in vacuo, resulting in 6.22 g crude product which was pure enough for further reactions.

### Intermediate 14

### 4-benzyl-1-(4-methyl-2H-1-benzopyran-2-one-8-yl)piperazine

To a stirred solution of methyl (triphenylphosphoranylidene)acetate (3.0 g, 8.98 mmol) in 50 ml of DMF was added Intermediate 13 (2.41 g, 7.48 mmol) in 20 ml of DMF. The reaction mixture was heated at 160°C for 17 hours and cooled down, poured into ethylacetate 5% sodiumbicarbonate. The mixture was extracted with ethylacetate, washed with water and the solvent was removed in vacuo. The crude product was purified by column chromatography (SiO₂ // diethylether : hexane = 2 : 1). Yield 1.75 g (60%) of the title compound.

### Intermediate 15

### 1 benzyl-2,6-dimethyl-4-(2-methoxyphenyl)piperazine

The title compound was prepared analog to the method described for 4-benzyl-1-(2-methoxyphenyl)piperazine (Intermediate 11) starting from 2,6-dimethyl-4-(2-methoxyphenyl)piperazine. Yield : 84%

### Intermediate 16

### 1-benzyl-2,6-dimethyl-4-(3-formyl-2-methoxyphenyl)piperazine

The title compound was prepared analog to the method described for 4-benzyl-1-(3-acetyl-2-methoxyphenyl)piperazine (Intermediate 12) by reaction of Intermediate 15 with N,N-dimethylformamide and sec-butyllithium in THF. Yield : 44%

### Intermediate 17

### 1-benzyl-2,6-dimethyl-4-(3-formyl-2-hydroxyphenyl)piperazine

The title compound was prepared analog to the method described for 4-benzyl-1-(3-acetyl-2-hydroxyphenyl)piperazine (Intermediate 13) starting from Intermediate 16. Yield : 55%

### Intermediate 18

### 1-benzyl-2,6-dimethyl-4-(3-acetamide-2H-1-benzopyran-2-one-8-yl)piperazine

To a stirred solution of Intermediate 17 (3.64 g, 11.2 mmol) in 25 ml of toluene was added N-acetylglycine (1.45 g, 12.3 mmol), sodiumacetate (3.03 g, 36.9 mmol) and acetic anhydride (2.33 ml, 22.4 mmol). The reaction mixture was heated at 100°C for 3 hours, cooled down and extracted with ethylacetate. The ethylacetate layer was washed with 5% sodiumbicarbonate, water, dried and the solvent was removed in vacuo. The crude product was purified by column chromatography (SiO₂ // diethylether : hexane = 1:1). Yield 1.57 g (35%) of the title compound.

### Intermediate 19

### 2,6-dimethyl-4-(3-acetamide-2H-1-benzopyran-2-one-8-yl)piperazine

The title compound was prepared according to the method described for 1-[4-methyl-2H-1-benzopyran-2-one-8-yl]-piperazine (Example 13) starting from Intermediate 18. Yield 40%.

### Intermediate 20

### 4-benzyl-1-(2-methoxy-5-methylphenyl)piperazine

To a stirred solution of N-benzylpiperazine (29.5 ml, 165 mmol) in 200 ml of dry THF was added at 0°C n-butyllithium (73.0 ml, 183 mmol (2.5 M)). The reaction mixture was stirred 30 minutes at 0°C and 1 hour at 30°C. At this time the mixture was cooled down to 0°C and 1,2-dimethoxy-5-methylbenzene (25.0 g, 165 mmol) was added, stirring was continued at roomtemperature for 15 hours. The reaction mixture was poured into ethylacetate and 5% sodiumbicarbonate and extracted with ethylacetate. The combined organic layers were washed with water, dried and the solvent was removed in vacuo. The crude product was purified by column chromatography (SiO₂ // diethylether : hexane = 1 : 1). Yield 30.3 g (62%) of the title compound.

### Intermediate 21

### 4-benzyl-1-(3-formyl-2-methoxy-5-methylphenyl)piperazine

The title compound was prepared analog to the method described for 4-benzyl-1-(3-acetyl-2-methoxyphenyl)piperazine (Intermediate 12) by reaction of Intermediate 20 with N,N-dimethylformamide and sec-butyllithium in THF.

### Intermediate 22

### 4-benzyl-1-(3-formyl-2-hydroxy-5-methylphenyl)piperazine

The title compound was prepared analog to the method described for 4-benzyl-1-(3-acetyl-2-hydroxyphenyl)piperazine (Intermediate 13) starting from Intermediate 21.

### Intermediate 23

### 4-benzyl-1-(3-acetamide-5-methyl-2H-1-benzopyran-2-one-8-yl)piperazine

The title compound was prepared according to the method described for 1-benzyl-2,6-dimethyl-4-(3-acetamide-2H-1-benzopyran-2-one-8-yl)piperazine (Intermediate 18) starting from Intermediate 22.

### Intermediate 24

### 1-(3-acetamide-5-methyl-2H-1-benzopyran-2-one-8-yl)piperazine

The title compound was prepared according to the method described for 1-[4-methyl-2H-1-benzopyran-2-one-8-yl]piperazine (Example 13) starting from Intermediate 23.

### Intermediate 25

### 4-benzyl-1-(3-propylamino-2H-1-benzopyran-2-one-8-yl)piperazine

To a stirred mixture of Intermediate 5 and sodium bicarbonate in dimethylformamide was added propyliodide. The mixture was stirred at 60°C for 17 hours under a nitrogen atmosphere.
The title compound was isolated by column chromatography.

### EXAMPLE 1

### 1-[3-methyl-2H-1-benzopyran-2-one-8-yl]-piperazine, hydrochloride

To a stirred solution of 8-amino-3-methyl-2H-1-benzopyran-2-one (4.0 g, 22.8 mmol) in 250 ml of chlorobenzene was added bis-(2-chloroethyl)amine hydrochloride (4.46 g, 25.0 mmol). The mixture was heated at 130°C for 5 days while stirring under a nitrogen atmosphere. The reaction mixture was cooled to 90°C and diluted with ethylacetate. The solid was filtered off and washed with ethylacetate and the crude product was recrystallized from ethylacetate/ethanol. Yield : 3.2 g (51 %), m.p. 270-2°C.

### EXAMPLE 2

### 1-[3-methoxymethyl-2H-1-benzopyran-2-one-8-yl]-piperazine, hydrochloride

This compound was prepared by the method described for 1-[3-methyl-2H-1-benzopyran-2-one-8-yl]-piperazine hydrochloride (Example 1) using 8-amino-3-methoxymethyl-2H-1-benzopyran-2-one (2.6 g, 13.0 mmol) and bis-(2-chloroethyl)amine hydrochloride (2.68 g, 15.0 mmol) in 60 ml of chlorobenzene. The mixture was refluxed for 2 days. Yield 1.0 g (25%), m.p. 221-4°C

### EXAMPLE 3

### 1-[3-hydroxymethyl-2H-1-benzopyran-2-one-8-yl]-piperazine, hydrochloride

The title compound was prepared by the method described for 1-[3-methyl-2H-1-benzopyran-2-one-8-yl]-piperazine hydrochloride (Example 1) using 8-amino-3-hydroxymethyl-2H-1-benzopyran-2-one (0.72 g, 3.80 mmol) and bis-(2-chloroethyl)amine hydrochloride (0.75 g, 4.20 mmol) in 150 ml of chlorobenzene. The mixture was refluxed for 5 days. Yield : 0.15 g (14%), m.p. 270°C (dec).

### EXAMPLE 4

### 1-[3-acetamide-2H-1-benzopyran-2-one-8-yl]-piperazine, hydrochloride

This compound was prepared by the method described for 1-[3-methyl-2H-1-benzopyran-2-one-8-yl]-piperazine hydrochloride (Example 1) using 8-amino-3-acetamide-2H-1-benzopyran-2-one (5.46 g, 21.4 mmol), p-toluenesulfonic acid monohydrate (4.49 g, 23.6 mmol) and bis-(2-chloroethyl)amine hydrochloride (4.21 g, 23.6 mmol) in 200 ml of chlorobenzene. The mixture was refluxed for 4 days using a Dean-Stark water separator. Yield : 3.90 g (60%), m.p. 298-303°C (dec)

### EXAMPLE 5

### 1-[3-hydroxy-2H-1-benzopyran-2-one-8-yl]-piperazine, hydrochloride

Intermediate 2 (1.60 g, 4.29 mmol) was converted into the title compound 1-[3-hydroxy-2H-1-benzopyran-2-one-8-yl]-piperazine hydrochloride by debenzylation with H₂ and Pd/C in 150 ml of 96% ethanol at 40°C for 1 hour. After filtration over Hyflo the crude product was converted into its hydrochloride salt and then recrystallized from 100% ethanol. Yield : 1.20 g (75%), m.p. 297°C

### EXAMPLE 6

### 1-[3-methoxy-2H-1-benzopyran-2-one-8-yl]-piperazine, hydrochloride

The Intermediate 3 was converted into the title compound 1-[3-methoxy-2H-1-benzopyran-2-one-8-yl]-piperazine hydrochloride by debenzylation with H₂ and Pd/C in 150 ml 96% ethanol at 40°C for 3 hours. After filtration over hyflo the crude product was recrystallized from 100% ethanol. Yield : 0.90 g (73%), m.p. 279-81°C (dec)

### EXAMPLE 7

### 1-[3-methylamino-2H-1-benzopyran-2-one-8-yl]-piperazine, hydrochloride

Debenzylation of Intermediate 4 (0.80 g, 2.07 mmol) with H₂ and Pd/C in 100 ml 96% ethanol at room temperature for 2 days gave the title compound 1-[3-methylamino-2H-1-benzopyran-2-one-8-yl]-piperazine hydrochloride. Yield : 0.55 g (90%), m.p. > 250°C (dec)

### EXAMPLE 8

### 1-[3-amino-2H-1-benzopyran-2-one-8-yl]-piperazine, hydrochloride

Debenzylation of Intermediate 5 (0.200 g, 0.50 mmol) with H₂ and Pd/C in 25 ml of 96% ethanol at room temperature for 5 hours gave the title compound 1-[3-amino-2H-1-benzopyran-2-one-8-yl]-piperazine hydrochloride. Yield : 0.110 g (79%), m.p. 282-6°C (dec)

### EXAMPLE 9

### 1-[6-chloro-2H-1-benzopyran-2-one-8-yl]-piperazine, hydrochloride

To a stirred solution of 8-amino-6-chloro-2H-1-benzopyran-2-one (1.65 g, 4.50 mmol) in 15 ml of p-xylene was added bis-(2-chloroethyl)amine hydrochloride (0.88 g, 4.95 mmol). The mixture was heated at 160°C for 18 hours while stirring under a nitrogen atmosphere. The reaction mixture was cooled down and concentrated in vacuo. The resulting dark solid was purified by column chromatography (SiO₂ // CH₂Cl₂ : MeOH : NH₄OH = 84 : 15 : 1) and converted into its hydrochloride salt. Yield : 1.36 g (45%) of 1-[6-chloro-2H-1-benzopyran-2-one-8-yl]-piperazine hydrochloride, m.p. > 275°C (dec)

### EXAMPLE 10

### 1-[6-methyl-2H-1-benzopyran-2-one-8-yl]-piperazine, fumarate

8-amino-6-methyl-2H-1-benzopyran-2-one hydrochloride (3.00 g, 14.2 mmol) together with bis-(2-chloroethyl)amine hydrochloride (2.75 g, 15.4 mmol) and p-toluenesulfonic acid monohydrate (2.88 g, 15.3 mmol) in 35 ml of p-xylene and 35 ml of chlorobenzene was heated at refluxtemperature under a nitrogen atmosphere for 18 hours using a Dean-Stark water separator. The reaction mixture was cooled down and concentrated in vacuo. The dark residue was purified by flash column chromatography (SiO₂) using an eluent gradient (1. CH₂Cl₂ 2. CH₂Cl₂ : MeOH : NH₄OH = 92: 7.5 : 0.5). The so obtained crude product was converted into its fumarate and recrystallized (methanol). Yield : 1.76 g (34%), m.p. 210-3°C (dec)

### Example 11

### 4-isopropyl-1-[3-amino-2H-1-benzopyran-2-one-8-yl]-piperazine, fumarate

Intermediate 7 was hydrolyzed with methanol and sulfuric acid as described for 4-methyl-1-[3-amino-2H-1-benzopyran-2-one-8-yl]-piperazine hydrochloride (Example 14). The crude product was purified by column chromatography (SiO₂ // CH₂Cl₂ : MeOH : NH₄OH = 84 : 15 : 1.0). The so obtained product was converted into its fumarate, m.p. 165-7°C

### Example 12

### 4-allyl-1-[3-amino-2H-1-benzopyran-2-one-8-yl]-piperazine, ½ fumarate

Intermediate 8 was hydrolyzed with methanol and sulfuric acid as described for 4-methyl-1-[3-amino-2H-1-benzopyran-2-one-8-yl]-piperazine hydrochloride (Example 14). The crude product was converted into its fumarate, m.p. 110°C (dec.)

### Example 13

### 1-[4-methyl-2H-1-benzopyran-2-one-8-yl]-piperazine, fumarate

Intermediate 14 (1.75 g, 5.43 mmol) was debenzylated with Pearlman Catalyst in 75 ml of methanol under a hydrogen atmosphere at roomtemperature for 6 hours. The reaction mixture was filtered over Hyflo and the solvent was removed in vacuo. The residue was purified by column chromatography (SiO₂ // ethylacetate : methanol : ammonia = 83 : 15 : 2) and converted into its fumarate. Yield : 1.33 g (100%), m.p. 197-199°C.

### Example 14

### 4-methyl-1-[3-amino-2H-1-benzopyran-2-one-8-yl]-piperazine, hydrochloride

Intermediate 6 (0.50 g, 1.67 mmol) was added to a mixture of 50 ml of methanol and 0.5 ml sulfuric acid. The reaction mixture was heated at 50°C for 1 hour, cooled down and neutralized with a 5% sodiumbicarbonate solution. The methanol was removed in vacuo and the residue extracted with dichloromethane. This organic phase was dried and the solvent removed in vacuo. The residue was converted into its hydrochloride. Yield : 0.34 g (60%), m.p. 312°C (dec.)

### Example 15

### 1-[3-n-propyl-2H-1-benzopyran-2-one-8-yI]-piperazine, ½ fumarate

The title compound was prepared according to the method described for 1-[3-methyl-2H-1-benzopyran-2-one-8-yl]-piperazine, hydrochloride (Example 1) starting from 8-amino-3-n-propyl-2H-1-benzopyran-2-one (Intermediate 10). Yield 22%, m.p. 182-3°C.

### Example 16

### 3,5-dimethyl-1-[3-amino-2H-1-benzopyran-2-one-8-yl]-piperazine, fumarate

The title compound was obtained after hydrolyzation of Intermediate 19 according to the method described for 4-methyl-1-[3-amino-2H-1-benzopyran-2-one-8-yl]-piperazine hydrochloride (Example 14). Yield 94%, m.p. 280-1°C.

### Example 17

### 1-[3-amino-6-methyl-2H-1-benzopyran-2-one-8-yl]-piperazine, 1½ fumarate

The title compound was prepared from Intermediate 24 by the method described for 4-methyl-1-[3-amino-2H-1-benzopyran-2-one-8-yl]piperazine hydrochloride (Example 14). Yield: 98%, m.p. 163-5°C.

### Example 18

### 1-[3-n-propylamino-2H-1-benzopyran-2-one-8-yl]-piperazine, fumarate

The title compound was prepared from Intermediate 25 by the method described for 1-[3-methylamino-2H-1-benzopyran-2-one-8-yl]-piperazine, hydrochloride (Example 7),
m.p. 198-201°C.

### Example 19

### 1-[3-pyrrolidinyl-2H-1-benzopyran-2-one-8-yl]-piperazine, fumarate

This compound was prepared analog to the method described for 1-[3-n-propylamino-2H-1-benzopyran-2-one-8-yl]-piperazine (Example 18) starting from Intermediate 25 and 1,4-dibromobutane. m.p. 210-12°C

### Example 20

### 1-[3-pipendinyl-2H-1-benzopyran-2-one-8-yl]-piperazine, fumarate

This compound was prepared analog to the method described for 1-[3-n-propylamino-2H-1-benzopyran-2-one-8-yl]-piperazine (Example 18) starting from Intermediate 25 and 1,5-dibromopentane. m.p. 230-1°C

## Claims

1. Piperzinederivatives of the formula (1) 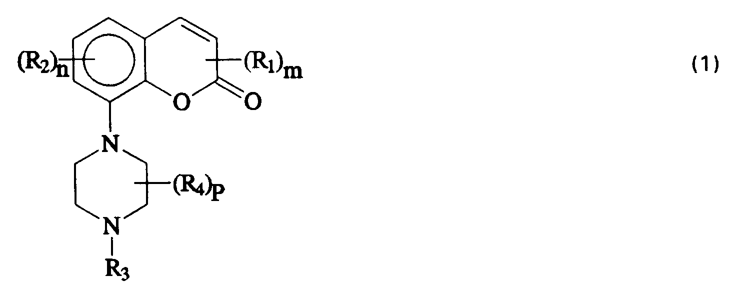 wherein
- R₁ is alkyl (1-4C), alkoxy (1-4C), hydroxy, alkoxy(1-4C)alkyl(1-4C), pyrrolidinyl, piperidinyl, morpholinyl, halogen, cyano, trifluoromethyl, amino, or mono- or disubstituted amino wherein the substituents are alkyl (1-4C), or alkyl(1-4C)carbonyl,
- m has the value 0, 1 or 2,
- R₂ is alkyl (1-4C), alkoxy (1-4C), halogen or trifluoromethyl,
- n is 0 or 1, on the understanding that m + n is at least 1,
- R₃ is hydrogen, alkyl (1-3C) or alkenyl (2-3C),
- R₄ is alkyl (1-4C), and
- p has the value 0,1 or 2,
and salts thereof with pharmaceutically acceptable acids.

2. Compounds as claimed in claim 1, characterized in that (R₂)ₙ and (R₄)ₚ represent hydrogen, R₃ has the meanings given in claim 1 and (R₁)ₘ is a substituent at position 3 selected from the group consisting of pyrrolidinyl, piperidinyl, morpholinyl, and amino and mono- or disubstituted amino wherein the substituents are alkyl (1-4C), or alkyl(1-4C)carbonyl.

3. Compound according to claim 2, characterized in that (R₁)ₘ is the group 3-amino, and R₃ is hydrogen, and the addition salts thereof with pharmaceutically acceptable acids.

4. A pharmaceutical composition, characterized in that it contains a compound as claimed in claim 1 as the active component, and pharmaceutically acceptable carrier and/or auxiliary materials.

5. A method for treating affections or diseases which result from disturbances in the central nervous system, characterized in that a composition as claimed in claim 5 is used.

6. A method for the preparation of piperazine derivatives, characterized in that a compound as claimed in claim 1 is prepared in a manner know per se for the synthesis of analogous compounds.

7. A method as claimed in claim 6, characterized in that a compound of the formula (2) 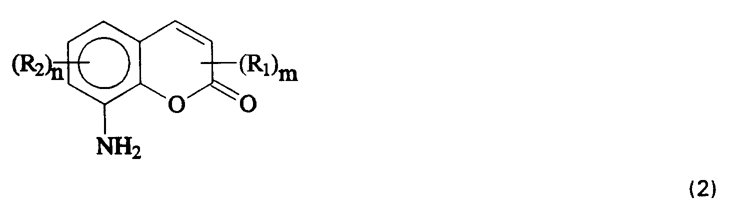 is reacted with a compound of the formula (3) 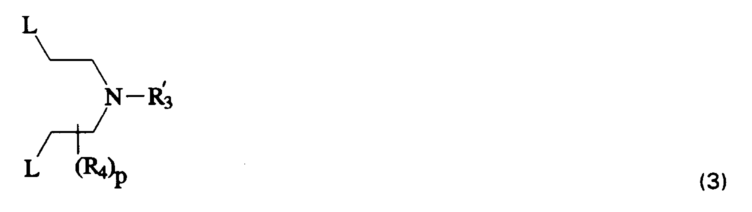 in which formulae R₁, R₂, R₄, m, n and p have the meanings given in claim 1, L is a leaving group, and R'₃ has the same meanings as R₃ or represents a group which protects the nitrogen atom.

8. A method as claimed in claim 6, characterized in that a compound of the formula (6) 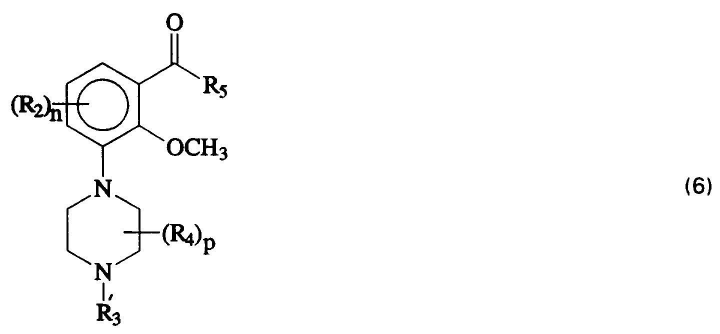 wherein R₂, R'₃, R₄, n and p have the meaning given in claim 7 and R₅ is hydrogen or methyl, is converted into a compound having formula (1) according to methods known per se for the synthesis of coumarins.
